# EUROPEAN PATENT SPECIFICATION

(11) **EP 3 930 776 B1**
(45) Date of publication and mention of the grant of the patent: **07.06.2023**
(21) Application number: 20713739.9
(22) Date of filing: 25.02.2020
(51) Int. Cl.: A61L 27/38, A61L 27/44, A61L 27/52, A61M 37/00, A61N 1/20, A61N 1/32, B82Y 5/00

(54) **MATERIAL AND SYSTEM FOR THE THERAPEUTIC TREATMENT OF JOINTS**
MATERIAL UND SYSTEM ZUR THERAPEUTISCHEN BEHANDLUNG VON GELENKEN
MATÉRIAU ET SYSTÈME POUR LE TRAITEMENT THÉRAPEUTIQUE DES ARTICULATIONS

(30) Priority: 25.02.2019 IT 201900002697
(43) Date of publication of application: 05.01.2022
(73) Proprietor: Vimex Spolka z Ograniczona Odowiedzialnoscia (VIMEX), 44-122 Gliwice (PL); H&D Wireless AB, 16451 Kista (SE); PLASMACHEM PRODUKTIONS UND HANDEL GMBH, 12489 Berlin (DE); Image Guided Therapy SA, 33600 Pessac (FR); Istituto Ortopedico Rizzoli, 40136 Bologna (IT); Bar Ilan University, 5920002 Ramat-Gan (IL); Scuola Superiore di Studi Universitari e di Perfezionamento Sant'Anna, 56127 Pisa (IT)
(72) Inventor: RICOTTI, Leonardo, 56037 Peccioli (IT); VANNOZZI, Lorenzo, 56024 San Miniato (PI) (IT); CAFARELLI, Andrea, 54033 Carrara (MS) (IT); NESSIM, Gilbert Daniel, 52900 Ramat Gan Israel (IL); LISIGNOLI, Gina, 40136 Bologna (IT); WECHSLER, Aharon, 6083016 Shoham (IL); DUMONT, Erik Jean-Claude, 33600 Pessac (FR); JOST, Carsten, 12435 Berlin (DE); GAPINSKI, Tomasz, 41-819 Zabrze (PL); BERGSTEN, Pär, 16955 Solna (SE); GABUSI, Elena, 40136 Bologna (IT); FINI, Milena, 40136 Bologna (IT); TSCHON, Matilde, 40136 Bologna (IT); RUSSO, Alessandro, 40136 Bologna (IT); ZAFFAGNINI, Stefano, 40136 Bologna (IT); MELICONI, Riccardo, 40136 Bologna (IT); FEDUTIK, Yirij, 12489 Berlin (DE); LENARTOWICZ, Krzysztof Stanislaw, 44-217 Rybnik (PL); JERNBERGER, Åke, 19267 Sollentuna (SE); SHACHAF, Yonatan, 3254701 Haifa (IL); ERIKSSON, Magnus, 135 33 Tyresö (SE)
(74) Representative: ABM Agenzia Brevetti & Marchi
(86) International application number: PCT/IB2020/051603
(87) International publication number: WO 2020/174395

(56) References cited:
- US-A1- 2012 121 712
- Ricotti: "ADMAIORA", , 1 January 2019 (2019-01-01), XP055633893, Retrieved from the Internet: URL:https://www.admaiora-project.com/wp-co ntent/uploads/2019/05/Admaiora-brochure-FI NAL-may-19.pdf [retrieved on 2019-10-18]
- JAICY JACOB ET AL: "Piezoelectric smart biomaterials for bone and cartilage tissue engineering", ENSHOU, SAISEI - INFLAMMATION AND REGENERATION, vol. 38, no. 1, 27 December 2018 (2018-12-27), XP055634014, JP ISSN: 1880-9693, DOI: 10.1186/s41232-018-0059-8
- AGATA PRZEKORA: "Current Trends in Fabrication of Biomaterials for Bone and Cartilage Regeneration: Materials Modifications and Biophysical Stimulations", INTERNATIONAL JOURNAL OF MOLECULAR SCIENCES, vol. 20, no. 2, 20 January 2019 (2019-01-20), page 435, XP055633897, DOI: 10.3390/ijms20020435

## Description

### Field of the invention

The present invention relates to the therapeutic treatment of joints.

In particular, the invention relates to a composite material and a treatment system for the regeneration of cartilage tissue.

### Description of the prior art

As known, osteoarthritis is currently the most common rheumatological disease: it causes severe motor disabilities and pain, which prevent regular daily life activities. This pathology mostly affects the elderly or obese population, although the cases found in sportsmen of young age following joint injuries are not negligible.

Osteoarthritis is a chronic degenerative disease and is manifested by symptoms that are difficult to diagnose in the initial stages, but which intensify quickly over time. If left untreated in the appropriate time and manner, the level of disability can gradually increase over time. At the level of the joint, due to this pathology, lesions on the cartilaginous surface and at the osteo-chondral interface can occur. The aggravation of the conditions can quickly lead to the final stage, which involves the implantation of a joint prosthesis.

In physiological conditions, the articular cartilage is responsible for the correct functioning of the joints, distributing the load (function of "shock absorber") and guaranteeing the necessary lubrication to avoid further problems from rubbing between tissues. Cartilage alterations can cause joint pain, given by the inability of the joint to support the load adequately.

In these early symptomatic phases, a preservation therapy of the joint structure is necessary. The great majority of this type of therapy is based on viscosupplements or platelet enriched plasma (PRP), which are able to promote a lubricating action or an anti-inflammatory effect on the compromised joint, thus ensuring a better patient condition.

However, both viscosupplements and PRP must be injected periodically into the joint, given that this type of treatment relieves the patient's condition only in the short term. The beneficial effects of these treatments are also very limited and highly dependent on the patient.

Once the degeneration of the cartilage tissue reaches a level of degeneration that can no longer be treated with the therapies mentioned above, osteoarthritis is treated surgically. Common practices are microfracture, autologous chondrocyte transplantation, autologous osteochondral transplantation and synthetic scaffolds.

However, even these systems appear to lose their effectiveness over time, often requiring an absolutely much more invasive intervention, such as the implantation of a knee prosthesis.

In addition, both the injections and the transplants mentioned above can lead to inflammations that are difficult to cure.

A further disadvantage of these therapies concerns the very high costs that the patient has to bear, especially in case of poor efficacy of the treatment, for which frequent reiteration is necessary.

Recently, some scientific studies have considered ultrasonic stimulation of piezoelectric nanomaterials for cell regeneration, in particular neuronal and muscle cells.

In US2012121712, for example, a method is described which provides for the induction of a non-invasive stimulation of neuronal cells, both in vitro and in vivo, through the use of piezoelectric nanovectors. Specifically, these are boron nitride nanotubes (BNNT) capable of being internalized by the cells and of converting a specific non-invasive external stimulus (ultrasound) into electrical inputs capable of stimulating the cells themselves. The nanotransducers are optionally coated with specific polymers. This method aims to reduce the high invasiveness typical of electrical cell stimulation (electrotherapy) methodologies that make use of electrodes to be inserted near the cellular tissues to be stimulated.

In addition, in the state of the art there are studies that take into consideration the ultrasonic stimulation of piezoelectric nanomaterials in order to treat and regenerate articular cartilage. Some examples are reported in "Piezoelectric smart biomaterials for bone and cartilage tissue engineering" by Jaicy Jacob et al. (https://inflammregen.biomedcentral.com/articles/10.1186/s4 1232-018-0059-8) and in "Current Trends in Fabrication of Biomaterials for Bone and Cartilage Regeneration: Materials Modifications and Biophysical Stimulations" by Agata Przekora (https: //www.mdpi.com/1422-0067/20/2/435).

However, the composite materials used in the state of the art do not optimize the distribution of the electric charges generated by the piezoelectric particles in the entire volume of the polymer matrix, making the cell regeneration process less effective and homogeneous, and typically do not have mechanical properties and optimal friction for cartilage applications.

### Summary of the invention

It is therefore a feature of the present invention to provide a system comprising a composite material for the therapeutic treatment of joints that allows to significantly slow down or even reverse the degenerative and inflammatory process affecting the articular cartilage, slowing down or avoiding the need to resort to a joint prosthesis.

It is also a feature of the present invention to provide such a system for avoiding the drawbacks, in terms of costs and efficiency, of articular therapies of the prior art.

It is also a feature of the present invention to provide such a system that exploits the known principle of ultrasonic stimulation of piezoelectric nanomaterials for the regeneration of cartilage tissues.

It is also a feature of the present invention to provide a method for the therapeutic treatment of joints which involves the use of this system and of this composite material.

These and other objects are achieved by a composite material arranged to treatment therapeutic of joints comprising:
- a biodegradable polymer matrix;
- a plurality of piezoelectric particles adapted to generate local electric charges in response to an external stimulation made by means of ultrasound, said plurality of piezoelectric particles being dispersed matrix;
- a plurality of stem cells dispersed in the biodegradable polymer matrix;
whose main feature is that in the biodegradable polymer matrix are also dispersed carbon-based particles.

According to another aspect of the invention, a system for the therapeutic treatment of joints is also claimed comprising:
- a composite material comprising:
   - a biodegradable polymer matrix;
   - a plurality of piezoelectric particles adapted to generate local electric charges in response to an external stimulation made by means of ultrasound, said plurality of piezoelectric particles being dispersed in the matrix;
   - a plurality of stem cells dispersed matrix;
- a releasing device arranged to deposit the composite material in a joint cavity at predetermined areas of the cartilage;
- a stimulator device arranged to emit ultrasound at a predetermined frequency, a predetermined intensity and for a predetermined time of application, in such a way that, when the device is located near a articulation wherein said composite material has been deposited, the ultrasound stimulate the plurality of piezoelectric particles;
whose main feature is that in the biodegradable polymer matrix are also dispersed carbon-based particles.

When the composite material is stimulated by means of ultrasound, the piezoelectric particles generate electric charge that have an chondrogenic effect on the stem cells, going to regenerate the cartilage and possibly having an anti-inflammatory effect.

In particular, the carbon-based particles allow to increase the mechanical resistance and to decrease the friction coefficient of the composite material, in addition to allowing a more effective distribution, within the matrix, of the electric charges generated by the piezoelectric particles.

In particular, the stem cells are selected from the group consisting of:
- autologous stem cells;
- heterologous stem cells;
- a combining the previous.

In particular, the piezoelectric particles are nanoparticles.

Advantageously, piezoelectric nanoparticles can have the form of "nanotubes", "nanowires", "nanorods", "nanospheres", "nanobelts", "nanowalls", "nanodisks", "nanoplates", "nanotripods", or others.

In particular, the piezoelectric nanoparticles are selected from the group consisting of:
- barium titanate particles;
- zinc oxide particles;
- piezoelectric polymer particles, such as PVDF or P (VDF-TrFE);
- KNN or NKN particles;
- alkaline niobate particles;
- PZT particles;
- boron nitride particles;
- PM PMN-PT particles;
- a combining the previous.

In particular, the biodegradable polymer matrix has viscosity set between 10 mPa*s and 10⁵ mPa*s.

In particular, the piezoelectric particles are previously subject to chemical functionalization of the surface and/or coating with chemical groups which facilitate the inclusion and dispersion of these particles in the polymer matrix and/or which improve their biocompatibility.

In particular, said biodegradable polymer matrix is a hydrogel.

The use of a composite material in the form of gel allows the deposit and the maintenance of piezoelectric particles and stem cells within defects that can be present on the surface of the cartilaginous tissue, remarkably increasing the efficiency of the treatment with respect to the prior art.

In particular, the carbon-based particles can be carbon nanotubes, or graphene, graphene oxide, reduced graphene oxide or other in the form of a single layer, laminar structures or other. These particles can also be chemically functionalized.

In particular, chondrocytes are also dispersed in the biodegradable polymer matrix.

Alternatively, other cells with paracrine action can be dispersed in the matrix with respect to the stem cells.

In particular, the matrix comprises biomolecules arranged for inducing the stem cells to evolve into chondrocytes.

In particular, the composite material is deposited at degradations and/or degenerations and/or defects of the cartilage.

Advantageously, a wearable device is also comprised comprising said stimulator device, said wearable device being configured to place the stimulator device near a joint.

Advantageously, a monitoring apparatus is also comprised arranged for monitoring the regeneration status of the cartilaginous tissue.

In particular, the monitoring apparatus comprises a device for ultrasound scanning.

Advantageously, a control unit is also comprised configured for:
- receiving data that correlate values of parameters characterizing the ultrasound with the speed and the quality of the cartilaginous tissue regeneration;
- setting the characterizing parameters on ranges of values that guarantee a greater speed and quality of the cartilaginous tissue regeneration.

In particular, the characterizing parameters are selected from the group consisting of:
- frequency of said ultrasound;
- intensity of said ultrasound;
- time of applying said ultrasound;
- duty cycle of said ultrasound;
- a combining the previous.

According to a further aspect of the invention, a method for the therapeutic treatment of joints is also claimed comprising the steps of:
- prearranging a composite material comprising:
   - a biodegradable polymer matrix;
   - a plurality of piezoelectric particles adapted to generate local electric charges in response to an external stimulation made by means of ultrasound, said plurality of piezoelectric particles being dispersed in the matrix;

   - a plurality of stem cells dispersed in the matrix;
- depositing the composite material in a joint cavity at predetermined areas of the cartilage;
- transmitting ultrasound at said joint cavity for stimulating the plurality of piezoelectric particles.

Advantageously, a step is also provided of application of a wearable device at the articulation, said wearable device comprising at least one ultrasound emitter.

In particular, a step is also provided of monitoring the articulation for monitoring the regeneration status of the cartilaginous tissue.

In particular, the step of monitoring is made by means of ultrasound scanning.

Advantageously, are also provided the steps of:
- collecting data that correlate values of parameters characterizing the ultrasound with the speed and the quality of the cartilaginous tissue regeneration;
- setting the characterizing parameters on ranges of values that guarantee a greater speed and quality of the cartilaginous tissue regeneration.

In particular, the characterizing parameters are selected from the group consisting of:
- frequency of said ultrasound;
- intensity of said ultrasound;
- time of applying said ultrasound;
- duty cycle of said ultrasound;
- a combining the previous.

### Brief description of the drawings

Further characteristic and/or advantages of the present invention are more bright with the following description of an exemplary embodiment thereof, exemplifying but not limitative, with reference to the attached drawings in which:
- Fig. 1 shows schematically the composite material according to the present invention;
- Fig. 2 shows the deposit of the composite material in the joint by the releasing device;
- Fig. 3 shows the wearable device arranged on the leg of a patient and a stimulator device adapted to emit ultrasound;
- Fig. 4 shows a possible flow-sheet of the operations made by the system according to the present invention;
- Fig. 5 shows a variant of the flow-sheet of Fig. 4, wherein a step of monitoring is further provided.

### Description of a preferred exemplary embodiment

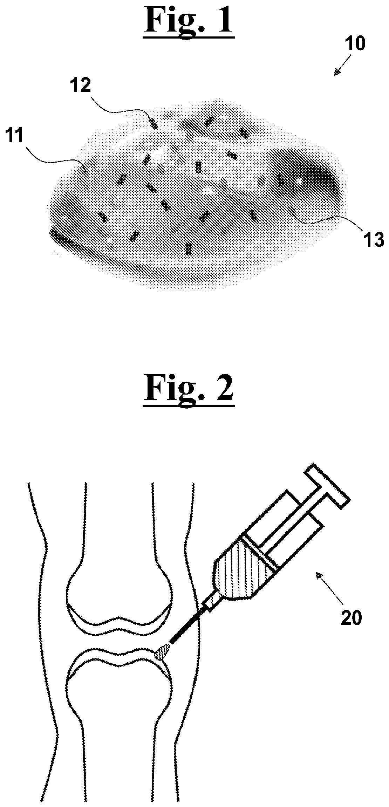

Fig. 1 shows an exemplary embodiment of the composite material 10, according to the present invention, comprising a biodegradable polymer matrix 11 in which a plurality of piezoelectric particles 12 and a plurality of stem cells 13 are dispersed, in addition to a plurality of carbon-based particles.

In particular, the carbon-based particles allow to increase the mechanical resistance and to decrease the friction coefficient of the composite material, in addition to allowing a more effective distribution, within the matrix, of the electric charges generated by the piezoelectric particles.

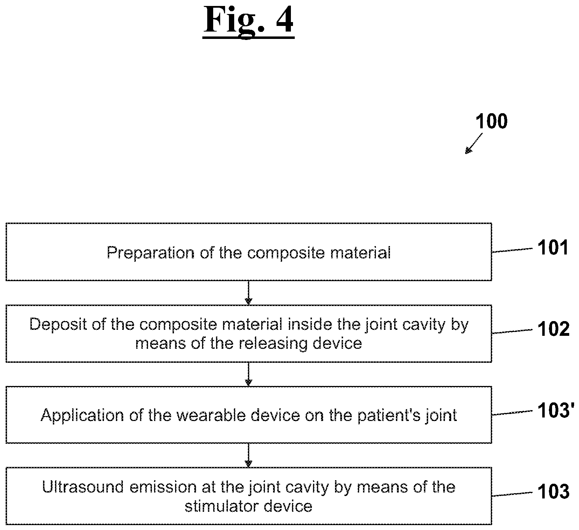

Figure 4 shows a possible flow chart 100 of the operations made by the system according to the present invention.

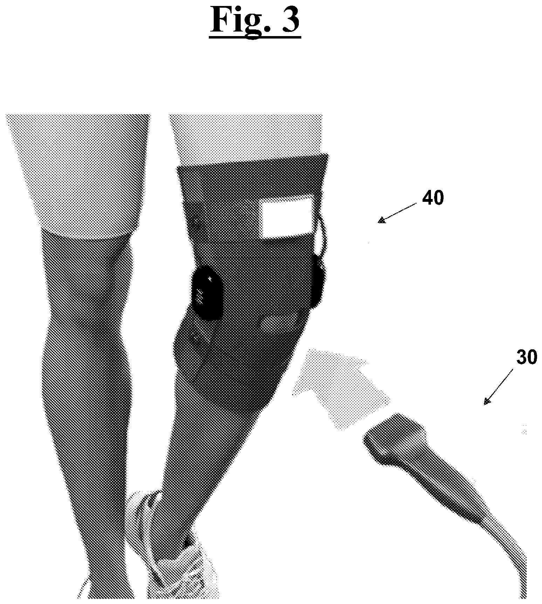

In particular, with reference even at Figs. 2 and 3, after having prepared the composite material 10 [101], the composite material 10 is adapted to be deposited by a releasing device 20, for example by injection, in a joint cavity at degradations and/or at defects of the cartilage of the articulation to treat [102].

When the composite material 10 is stimulated by means of ultrasound emitted by the stimulator device 30 provided by the present invention, the piezoelectric particles are stimulated and, consequently, generate electric charges which have a chondrogenic on the stem cells, going to regenerate the cartilage [103].

The system can furthermore provide a wearable device 40 that can contain the stimulator device 30 inside, in order to direct the ultrasound towards the joint cavity and stimulate the piezoelectric particles automatically at predetermined intervals [103'].

In the preferred exemplary embodiment of Fig. 1, the biodegradable polymer matrix 11 is, in particular, a hydrogel. This allows the material 10 to have enough density and viscosity for keeping the piezoelectric particles 12 and the stem cells 13 at close distance so as to increase their interaction. Furthermore, the form of hydrogel allows the composite material 10 to be injected in the joint by adequately filling the joint cavity and coming into contact with each lesion of the cartilage, then remaining in a stable position.

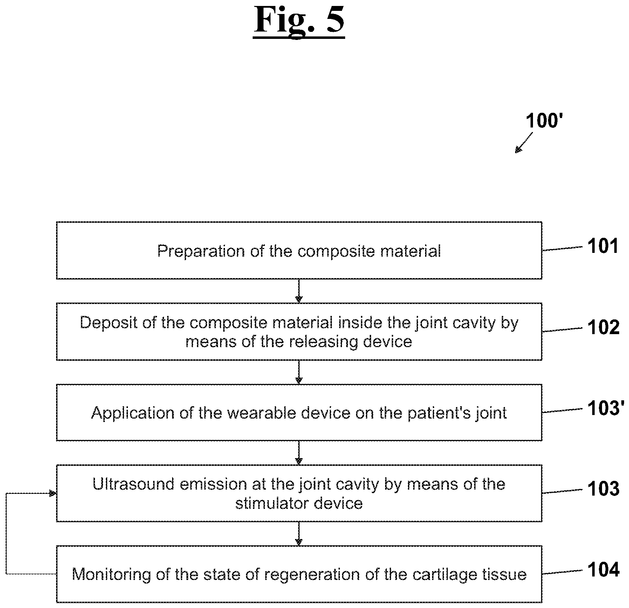

Figure 5 shows a variant of the flow-sheet of Fig. 4, wherein a step of monitoring the regeneration status of the cartilaginous tissue is further provided, , operated by a control unit. On the basis of this monitoring, the control unit can then subsequently modify the characterizing parameters of the ultrasounds in order to guarantee greater speed and quality of the cartilage tissue regeneration.

## Claims

1. A system for the therapeutic treatment of joints comprising:
- a composite material (10) comprising:
- a biodegradable polymer matrix (11);
- a plurality of piezoelectric particles (12) adapted to generate local electric charges in response to an external stimulation made by means of ultrasound, said plurality of piezoelectric particles (12) being dispersed in said biodegradable polymer matrix;
- a plurality of stem cells (13) dispersed in said biodegradable polymer matrix;
- a releasing device (20) arranged to deposit said composite material (10) in a joint cavity at predetermined areas of the cartilage;
- a stimulator device (30) arranged to emit ultrasound at a predetermined frequency, a predetermined intensity and for a predetermined time of application, in such a way that, when said device is located near a articulation wherein said composite material (10) has been deposited, said ultrasound stimulate said plurality of piezoelectric particles (12);
said system **characterized in that** in said biodegradable polymer matrix (11) are also dispersed carbon-based particles.

2. The system for the therapeutic treatment of articulations, according to claim 1, wherein a wearable device (40) is also provided comprising said stimulator device (30), said wearable device (40) being configured for arranging said stimulator device (30) near a joint.

3. The system for the therapeutic treatment of articulations, according to claim 1, wherein a monitoring apparatus is also comprised arranged for monitoring the regeneration status of the cartilaginous tissue.

4. The system for the therapeutic treatment of articulations, according to claim 3, where a control unit is also comprised configured for:
- collecting data that correlate values of parameters characterizing said ultrasound with speed and quality of the cartilaginous tissue regeneration;
- setting said characterizing parameters on ranges of values that guarantee greater speed and quality of the cartilaginous tissue regeneration.

5. The system for the therapeutic treatment of articulations, according to claim 4, wherein said characterizing parameters are selected from the group consisting of:
- frequency of said ultrasound;
- intensity of said ultrasound;
- time of applying said ultrasound;
- duty cycle of said ultrasound;
- a combining the previous.

6. A composite material (10) arranged to the therapeutic treatment of joints, said composite material (10) comprising:
- a biodegradable polymer matrix (11);
- a plurality of piezoelectric particles (12) adapted to generate local electric charges in response to an external stimulation made by means of ultrasound, said plurality of piezoelectric particles (12) being dispersed in said biodegradable polymer matrix;
- a plurality of stem cells (13) dispersed in said biodegradable polymer matrix (11);
said composite material (10) **characterized in that** in said biodegradable polymer matrix (11) are also dispersed carbon-based particles.

7. The composite material (10), according to claim 6, wherein said biodegradable polymer matrix is a hydrogel.

8. The composite material (10), according to claim 6, wherein in said biodegradable polymer matrix are also dispersed chondrocytes.

9. The composite material (10), according to claim 6, wherein said biodegradable polymer matrix comprises biomolecules configured for inducing said stem cells (13) to evolve into chondrocytes.

## Patentansprüche

1. System zur therapeutischen Behandlung von Gelenken, umfassend:
- ein Verbundmaterial (10), das Folgendes umfasst:
- eine biologisch abbaubare Polymermatrix (11);
- eine Vielzahl von piezoelektrischen Partikeln (12), die angeordnet ist, um lokale elektrische Ladungen als Reaktion auf eine externe Stimulation zu erzeugen, die mittels Ultraschall durchgeführt wird, wobei die Vielzahl von piezoelektrischen Partikeln (12) in der biologisch abbaubaren Polymermatrix dispergiert ist;
- eine Vielzahl von Stammzellen (13), die in der biologisch abbaubaren Polymermatrix dispergiert ist;
- eine Freisetzungsvorrichtung (20), die angeordnet ist, um das Verbundmaterial (10) in einem Gelenkhohlraum an vorbestimmten Bereichen des Knorpels abzulegen;
- eine Stimulatorvorrichtung (30), die angeordnet ist, um Ultraschall mit einer vorbestimmten Frequenz, einer vorbestimmten Intensität und für eine vorbestimmte Anwendungszeit derart auszusenden, dass, wenn sich die Vorrichtung in der Nähe einer Gelenkverbindung befindet, wobei das Verbundmaterial (10) abgelegt wurde, der Ultraschall die Vielzahl von piezoelektrischen Partikeln (12) stimuliert;
wobei das System **dadurch gekennzeichnet ist, dass** in der biologisch abbaubaren Polymermatrix (11) auch Partikel auf Kohlenstoffbasis dispergiert sind.

2. System zur therapeutischen Behandlung von Gelenkverbindungen nach Anspruch 1, wobei auch eine tragbare Vorrichtung (40) bereitgestellt ist, die die Stimulatorvorrichtung (30) umfasst, wobei die tragbare Vorrichtung (40) zum Anordnen der Stimulatorvorrichtung (30) in der Nähe eines Gelenks konfiguriert ist.

3. System zur therapeutischen Behandlung von Gelenkverbindungen nach Anspruch 1, wobei auch eine Überwachungseinrichtung umfasst ist, die zum Überwachen des Regenerationsstatus des Knorpelgewebes angeordnet ist.

4. System zur therapeutischen Behandlung von Gelenkverbindungen nach Anspruch 3, wobei auch eine Steuereinheit umfasst ist, die konfiguriert ist zum:
- Sammeln von Daten, die Werte von Parametern, die den Ultraschall charakterisieren, mit einer Geschwindigkeit und Qualität der Knorpelgeweberegeneration korrelieren;
- Einstellen der charakterisierenden Parameter auf Wertebereiche, die eine höhere Geschwindigkeit und Qualität der Knorpelgeweberegeneration garantieren.

5. System zur therapeutischen Behandlung von Gelenkverbindungen nach Anspruch 4, wobei die charakterisierenden Parameter aus der Gruppe ausgewählt sind, die besteht aus:
- Frequenz des Ultraschalls;
- Intensität des Ultraschalls;
- Anwendungszeit des Ultraschalls;
- Arbeitszyklus des Ultraschalls;
- einer Kombination der Vorstehenden.

6. Verbundmaterial (10), das zur therapeutischen Behandlung von Gelenken angeordnet ist, wobei das Verbundmaterial (10) umfasst:
- eine biologisch abbaubare Polymermatrix (11);
- eine Vielzahl von piezoelektrischen Partikeln (12), die angeordnet ist, um lokale elektrische Ladungen als Reaktion auf eine externe Stimulation zu erzeugen, die mittels Ultraschall durchgeführt wird, wobei die Vielzahl von piezoelektrischen Partikeln (12) in der biologisch abbaubaren Polymermatrix dispergiert ist;
- eine Vielzahl von Stammzellen (13), die in der biologisch abbaubaren Polymermatrix (11) dispergiert ist;
wobei das Verbundmaterial (10) **dadurch gekennzeichnet ist, dass** in der biologisch abbaubaren Polymermatrix (11) auch Partikel auf Kohlenstoffbasis dispergiert sind.

7. Verbundmaterial (10) nach Anspruch 6, wobei die biologisch abbaubare Polymermatrix ein Hydrogel ist.

8. Verbundmaterial (10) nach Anspruch 6, wobei in der biologisch abbaubaren Polymermatrix auch Chondrozyten dispergiert sind.

9. Verbundmaterial (10) nach Anspruch 6, wobei die biologisch abbaubare Polymermatrix Biomoleküle umfasst, die dazu konfiguriert sind, die Stammzellen (13) zu veranlassen, sich zu Chondrozyten zu entwickeln.

## Revendications

1. Système destiné au traitement thérapeutique des articulations comprenant :
- un matériau composite (10) comprenant :
- une matrice polymère biodégradable (11) ;
- une pluralité de particules piézoélectriques (12) adaptées à la génération de charges électriques locales en réponse à une stimulation externe effectuée au moyen d'ultrasons, ladite pluralité de particules piézoélectriques (12) étant dispersées dans ladite matrice polymère biodégradable ;
- une pluralité de cellules souches (13) dispersées dans ladite matrice polymère biodégradable ;
- un dispositif de libération (20) servant à déposer ledit matériau composite (10) dans une cavité articulaire au niveau de zones prédéfinies du cartilage ;
- un dispositif de stimulation (30) servant à émettre des ultrasons à une fréquence prédéfinie, une intensité prédéfinie et pendant un temps d'application prédéfini, de sorte que, lorsque ledit dispositif se trouve à proximité d'une articulation dans laquelle ledit matériau composite (10) a été déposé, lesdits ultrasons stimulent ladite pluralité de particules piézoélectriques (12) ;
ledit système **étant caractérisé en ce que** dans ladite matrice polymère biodégradable (11) sont également dispersées des particules à base de carbone.

2. Système destiné au traitement thérapeutique des articulations, selon la revendication 1, un dispositif portatif (40) étant également pourvu comprenant ledit dispositif de stimulation (30), ledit dispositif portatif (40) étant conçu pour disposer ledit dispositif de stimulation (30) à proximité d'une articulation.

3. Système destiné au traitement thérapeutique des articulations, selon la revendication 1, un appareil de surveillance étant également compris servant à surveiller l'état de régénération du tissu cartilagineux.

4. Système destiné au traitement thérapeutique des articulations, selon la revendication 3, une unité de commande étant également comprise conçue pour :
- collecter des données qui corrèlent des valeurs de paramètres de caractérisation desdits ultrasons avec la vitesse et la qualité de la régénération du tissu cartilagineux ;
- régler lesdits paramètres caractéristiques sur des plages de valeurs qui garantissent de plus grandes vitesse et qualité de la régénération du tissu cartilagineux.

5. Système destiné au traitement thérapeutique des articulations, selon la revendication 4, lesdits paramètres de caractérisation étant choisis dans le groupe constitué par :
- la fréquence desdits ultrasons ;
- l'intensité desdits ultrasons ;
- le temps d'application desdits ultrasons ;
- le cycle d'utilisation desdits ultrasons ;
- une combinaison des éléments précédents.

6. Matériau composite (10) servant au traitement thérapeutique des articulations, ledit matériau composite (10) comprenant :
- une matrice polymère biodégradable (11) ;
- une pluralité de particules piézoélectriques (12) adaptée à la génération de charges électriques locales en réponse à une stimulation externe effectuée au moyen d'ultrasons, ladite pluralité de particules piézoélectriques (12) étant dispersée dans ladite matrice polymère biodégradable ;
- une pluralité de cellules souches (13) dispersée dans ladite matrice polymère biodégradable (11);
ledit matériau composite (10) **étant caractérisé en ce que** dans ladite matrice polymère biodégradable (11) sont également dispersées des particules à base de carbone.

7. Matériau composite (10), selon la revendication 6, ladite matrice polymère biodégradable étant un hydrogel.

8. Matériau composite (10), selon la revendication 6, dans ladite matrice polymère biodégradable étant également dispersés des chondrocytes.

9. Matériau composite (10), selon la revendication 6, ladite matrice polymère biodégradable comprenant des biomolécules conçues pour induire l'évolution desdites cellules souches (13) en chondrocytes.
